# EUROPÄISCHE PATENTSCHRIFT

(11) **EP 3 082 962 B1**
(45) Veröffentlichungstag und Bekanntmachung des Hinweises auf die Patenterteilung: **06.02.2019**
(21) Anmeldenummer: 14799785.2
(22) Anmeldetag: 19.11.2014
(51) Int. Cl.: A61Q 5/06, A61Q 5/08, A61Q 5/10, A61K 8/73, A61K 8/04

(54) **OXIDATIONSFÄRBEMITTEL MIT VERRINGERTER HAARSCHÄDIGUNG**
OXIDATION DYE WITH REDUCED HAIR DAMAGE
AGENTS DE TEINTURE OXYDANTS ABIMANT PEU LES CHEVEUX

(30) Priorität: 19.12.2013 DE 102013226587
(43) Veröffentlichungstag der Anmeldung: 26.10.2016
(73) Patentinhaber: Henkel AG & Co. KGaA, 40589 Düsseldorf (DE)
(72) Erfinder: WESER, Gabriele, 41472 Neuss (DE); SCHUMACHER, Ulrike, 40589 Düsseldorf (DE)
(86) Internationale Anmeldenummer: PCT/EP2014/074968
(87) Internationale Veröffentlichungsnummer: WO 2015/090812

(56) Entgegenhaltungen:
- EP-A2- 0 962 218
- EP-A2- 2 345 400
- WO-A1-2013/069166
- WO-A1-2013/069168
- WO-A2-2013/013863

## Beschreibung

Gegenstand der vorliegenden Anmeldung sind Oxidationsfärbe- und/oder -aufhellmittel für keratinische Fasern, die gegenüber konventionellen Oxidationsfärbemitteln mit einer verringerten Haarschädigung einhergehen. Weiterhin betrifft die vorliegende Anmeldung ein entsprechendes Kit zur oxidativen Färbung oder Aufhellung von keratinhaltigen Fasern sowie ein Färbe- und/oder Aufhellverfahren für keratinische Fasern unter Zuhilfenahme der erfindungsgemäßen Mittel.

Für dauerhafte, intensive Färbungen mit entsprechenden Echtheitseigenschaften werden so genannte Oxidationsfärbemittel verwendet. Oxidative Färbemittel bestehen üblicherweise aus zwei Komponenten: die eine Komponente enthält üblicherweise Oxidationsfarbstoffvorprodukte, so genannte Entwicklerkomponenten und Kupplerkomponenten. Die Entwicklerkomponenten bilden unter dem Einfluss von Oxidationsmitteln, insbesondere Wasserstoffperoxid, die der ersten Komponenten erst kurz vor der Applikation auf das Haar beigemischt werden, oder von Luftsauerstoff untereinander oder unter Kupplung mit einer oder mehreren Kupplerkomponenten die eigentlichen Farbstoffe aus. Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt. Als Kupplerkomponenten werden in der Regel m-Phenylendiaminderivate, Naphthole, Resorcin und Resorcinderivate, Pyrazolone, m-Aminophenole und substituierte Pyridinderivate verwendet. Die Oxidationsfärbemittel zeichnen sich durch hervorragende, lang anhaltende Färbeergebnisse aus.

Konventionelle oxidative Färbemittel besitzen zur Stabilisierung der Farbstoffvorprodukte während der Lagerung und zur Reaktionsbeschleunigung während der oxidativen Anwendung einen stärker alkalischen pH-Wert, der deutlich über 9,0 liegt und mit Alkalisierungsmitteln, wie Alkanolaminen, Ammoniak oder anorganischen Basen, eingestellt wird. Insbesondere Ammoniak ermöglicht dabei gute Färbeergebnisse, offenbart jedoch aufgrund seines Geruchs und Reizpotenzials für Haut und Schleimhäute auch Nachteile für den Anwender. Das Alkalisierungsmittel führt zu einem Aufquellen der keratinischen Faser, wodurch die Farbstoffvorprodukte gut in das Haar eindringen können. Allerdings wird durch den alkalischen pH-Wert auch die schädigende Wirkung des Oxidationsmittels auf die Haarstruktur verstärkt. Daher liegen besondere Bemühungen auf der Entwicklung leistungsfähiger oxidativer Färbemittel, die einen möglichst niedrigen pH-Wert aufweisen, also nur einen geringen Gehalt an Alkalisierungsmitteln erforderlich machen, damit die Schädigung der Haarstruktur durch das Oxidationsmittel minimiert werden kann.

Kommerzielle Oxidationsfärbemittel, insbesondere solche, die im Einzelhandel verkauft und vom Kunden bzw. von der Kundin zu Hause angewendet werden, werden üblicherweise mit einer Standardgebrauchsanweisung versehen, die eine bestimmte Applikationsdauer empfiehlt, während der das Färbemittel auf dem Haar verbleiben soll. Diese Applikationsdauer wurde vom Hersteller in umfangreichen Probandentests ermittelt und richtet sich eher nach den Probanden mit schwerer färbbarem bzw. schwerer aufhellbarem Haar, bei denen die Farbe erst nach längerer Einwirkzeit auf das Haar aufzieht bzw. die der gewünschte Grad an Aufhellung längerer Einwirkzeit erzielt wird. Anwender mit leichter zu färbendem Haar könnten demnach die empfohlene Standard-Applikationsdauer durchaus abkürzen und dennoch ein gutes Färbe- oder Aufhellergebnis erzielen. Um die Schädigung der Haarstruktur noch weiter zu reduzieren, wäre es daher wünschenswert, die Applikationsdauer auf die Zeit zu begrenzen, nach der die gewünschte Haarfarbe erzielt wurde. Um dies zu erkennen, wäre es günstig, dass die Färbekomponente wie auch die Anwendungsmischung, also die Mischung aus Färbekomponente und Oxidationsmittel transparent sind.

Eine weitere Anforderung an oxidative Färbe- und/oder Aufhellmittel besteht darin, dass die Anwendungsmischung aus Farbstoffkomponenten und Oxidationsmittelkomponente ausreichend zähflüssig ist, damit sie bequem auf die Haare aufgetragen werden und während der gesamten Anwendungsdauer verbleiben kann und dabei nicht tropft oder verläuft.

EP 962218A2 offenbart ein Verfahren zur oxidativen Haarfärbung, das Haar schonend ist und eine Färbung mit verbesserter Deckkraft, Intensität und Stabilität bewirkt, wobei zunächst ein übliches Oxidationsfärbemittel mit einem pH-Wert von 9,5 bis 9,8 und nach einer Einwirkzeit von 5 bis 30 Minuten ein Säure-haltiges Gel mit einem pH-Wert von 2 - 6 auf das Haar aufgetragen wird, woraufhin das so auf einen pH von 5 bis 8 verdünnte Färbemittel für weitere 15 bis 20 Minuten einwirkt und dann abgespült wird.

EP 2345400A2 beschäftigt sich mit oxidativen Aufhell- und/oder Blondiermitteln, die bestimmte nichtionische Tenside und Carbonsäureesteröle enthalten und das Haar schützen und eine verringerte Schädigung des Haares bewirken, dem Haar erhöhte Elastizität und Geschmeidigkeit sowie einen verbesserten Glanz verleihen und Kopfhautirritationen vermindern, ohne die Aufhellleistung der Mittel zu beeinträchtigen. EP 2345400A2 offenbart keine Hydrogelbildner.

WO 2013/069168A1 und WO 2013/069166A1 offenbaren 2-Komponenten-Oxidationsfärbe- oder -aufhellmittel, die als Schaum aufgetragen werden, keinen starken Ammoniakgeruch, aber dennoch ein adäquates Bleich- oder Färbevermögen aufweisen und mindestens 20 Gew.-% Fettsubstanz und weitere obligatorische Inhaltsstoffe, wie Schaumstabilisierer und ausgewählte Tenside, enthalten. WO 2013/013863A2 offenbart oxidative Farbveränderungsmittel für die treibgasfreie Schaumapplikation mit verbesserter Schaumstabilität und verbesserten Färbeeigenschaften. Aufgabe der vorliegenden Erfindung war es daher, oxidative Färbe- und/oder Aufhellmittel für keratinische Fasern, insbesondere Haare, bereitzustellen, die eine möglichst geringe Haarschädigung bewirken, insbesondere bei mit konventionellen stark alkalischen Färbemitteln vergleichbar guten Färbe- und/oder Aufhellergebnissen und insbesondere bei gutem Aufziehvermögen der Färbung auf die keratinischen Fasern. Eine weitere Aufgabe der vorliegenden Erfindung war es, transparente oxidative Färbe- und/oder Aufhellmittel bereitzustellen.

Es wurde überraschenderweise gefunden, dass oxidative Färbe- und/oder Aufhellmittel, die als, bevorzugt transparentes, Hydrogel vorliegen und einen pH-Wert im Bereich von 7,0 bis 8,8 aufweisen und die bevorzugt nach Vermischen mit einer sauer eingestellten Oxidationsmittelzubereitung eine Anwendungsmischung mit einem pH-Wert im Bereich von 6,0 bis 7,3 ergeben, eine intensive Färbung bzw. Aufhellung der Fasern ermöglichen und dennoch mit einer verringerten Haarschädigung einhergehen.

Ein erster Gegenstand der vorliegenden Anmeldung ist ein Mittel zum Färben und/oder Aufhellen von keratinischen Fasern, enthaltend
- 80 - 93 Gew.-%, bezogen auf das Mittel, Wasser oder einer Mischung aus Wasser und mindestens einem wasserlöslichen Polyol, wobei mindestens 30 Gew.-% Wasser, bezogen auf das Mittel, enthalten sind, weiterhin
- als Hydrogel bildendes Polymere Xanthan und mindestens ein vernetztes Acrylsäure-Homopolymer weiterhin
- mindestens ein Alkalisierungsmittel, weiterhin
- mindestens einen Lösevermittler, ausgewählt aus Fettalkoholglycolethern, Fettalkoholen, Fettsäureglycerinestern und Fettsäuresorbitanestern, die jeweils ethoxyliert und optional propoxy liert sind,
- optional mindestens ein Oxidationsfarbstoffvorprodukt und/oder mindestens einen direktziehenden Farbstoff,
- wobei das Mittel einen pH-Wert im Bereich von 7,0 bis 8,8, gemessen bei 22°C, aufweist,
- als Hydrogel vorliegt und
- gekennzeichnet ist durch eine Trübung im Bereich von null bis 80 NTU, bevorzugt von maximal 60 NTU, besonders bevorzugt von maximal 50 NTU, außerordentlich bevorzugt von maximal 40 NTU, jeweils gemessen bei 22°C gemäß DIN EN ISO 7027-C2.

Die erfindungsgemäßen Mittel liegen als Hydrogel vor und enthalten dementsprechend 80 - 93 Gew.-% eines wässrigen Trägers, der entweder allein aus Wasser besteht oder aber aus einer Mischung aus Wasser und mindestens einem wasserlöslichen Polyol, wobei aber mindestens 30 Gew.-% Wasser, bezogen auf das Mittel, enthalten sind.

Erfindungsgemäß wird unter einem wasserlöslichen Polyol ein Polyol mit einer Löslichkeit von wenigstens 5 Gew.-% bei 20 °C verstanden, das heißt, dass wenigstens 5 g des Polyols in 95 g Wasser bei 20 °C löslich sind.

Bevorzugte wasserlösliche Polyole sind ausgewählt aus mindestens einem wasserlöslichen mehrwertigen C₂ - C₉-Alkanol mit 2 - 6 Hydroxylgruppen und/oder mindestens einem wasserlöslichen Polyethylenglycol mit 3 - 20 Ethylenoxid-Einheiten sowie Mischungen hiervon. Bevorzugt sind diese Polyole ausgewählt aus 1,2-Propylenglycol, 1,3-Propylenglycol, 2-Methyl-1,3-propandiol, Glycerin, 1,2-Butylenglycol, 1,3-Butylenglycol, 1,4-Butylenglycol, 1,2-Pentandiol, 1,5-Pentandiol, 1,2-Hexandiol, 1,6-Hexandiol, 2-Methyl-2,4-pentandiol, 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebige Isomeren-Gemische von cis- und trans-1,4-Dimethylolcyclohexan, PEG-3, PEG-4, PEG-6, PEG-7, PEG-8, PEG-9, PEG-10, PEG-12, PEG-14, PEG-16, PEG-18 und PEG-20 sowie Mischungen hiervon. Besonders bevorzugte wasserlösliche Polyole sind ausgewählt aus 1,2-Propylenglycol, Glycerin, 1,3-Butylenglycol, 1,6-Hexandiol, Dipropylenglycol und PEG-8, sowie Mischungen hiervon. Außerordentlich bevorzugt ist 1,2-Propylenglycol.

Das mindestens eine wasserlösliche Polyol ist bevorzugt in einer Gesamtmenge von 0,5 - 63 Gew.-%, besonders bevorzugt in einer Gesamtmenge von 1 - 50 Gew.-%, weiter besonders bevorzugt in einer Gesamtmenge von 2 - 30 Gew.-%, außerordentlich bevorzugt in einer Gesamtmenge von 4 - 10 Gew.-%, jeweils bezogen auf das Gewicht des erfindungsgemäßen Mittels.

Als zweite erfindungswesentliche Komponente enthalten die erfindungsgemäßen Mittel als Hydrogel bildendes Polymere Xanthan und mindestens ein vernetztes Acrylsäure-Homopolymer.

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen. Polymere werden durch Polymerisation von einem Monomertyp oder durch Polymerisation von verschiedenen, strukturell voneinander unterschiedlichen Monomertypen hergestellt. Wird das Polymer durch Polymerisation eines Monomertyps hergestellt, spricht man von Homo-Polymeren. Werden strukturell unterschiedliche Monomertypen in der Polymerisation eingesetzt, spricht der Fachmann von Copolymeren. Das maximale Molekulargewicht des Polymers hängt vom Polymerisationsgrad (Anzahl der polymerisierten Monomere) ab und wird durch die Polymerisationsmethode mitbestimmt. Im Sinne der vorliegenden Erfindung ist es bevorzugt, wenn das Molekulargewicht der verwendeten Polymere 100.000 bis 10⁷ g/mol, bevorzugt 200.000 bis 5.10⁶ g/mol und besonders bevorzugt 500.000 bis 1·10⁶ g/mol beträgt.

Eines der Hydrogel bildenden Polymere ist ausgewählt aus vernetzten Homopolymeren von Acrylsäure, insbesondere solchen mit der INCI-Bezeichnung Carbomer.

Mindestens ein Polysaccharid, ausgewählt aus Xanthan, Cellulose, Celluloseethern, vor allem Hydroxyalkylcellulosen, insbesondere Hydroxypropylcellulose, Hydroxypropylmethylcellulose, Hydroxyethylcellulose, Carboxymethylcellulose, Hydroxybutylmethylcellulose, Methylhydroxyethylcellulose, Sclerotium Gum, Succinoglucanen, Polygalactomannanen, insbesondere Guar-Gums und Johannisbrotkernmehl (Locust Bean Gum), insbesondere Guar-Gum und Locust Bean Gum selbst und den nichtionischen Hydroxyalkylguarderivaten und Johannisbrotkernmehl-Derivaten, wie Hydroxypropylguar, Carboxymethylhydroxypropylguar, Hydroxypropylmethylguar, Hydroxyethylguar und Carboxymethylguar, weiterhin Pectinen, Agar, kappa-Carrageen, iota-Carrageen, Traganth, Gummi arabicum, Karayagummi, Taragummi, Gellan, Gelatine, Casein, Propylenglycolalginat, Alginsäuren und deren Salze, insbesondere Natriumalginat, Kaliumalginat und Calciumalginat, sowie Mischungen hiervon, ist bevorzugt in einer Gesamtmenge von 0,2 bis 3 Gew.-%, besonders bevorzugt 0,5 - 2 Gew.-%, außerordentlich bevorzugt 0,8 bis 1,5 Gew.-%, jeweils bezogen auf das Gewicht des erfindungsgemäßen Mittels, enthalten, wobei zwingend Xanthan enthalten ist.

Das mindestens eine Polymer oder Copolymer der Acrylsäure und/oder der Methacrylsäure und/oder der 2-Methyl-2[(1-oxo-2-propenyl)amino]-1-propansulfonsäure, ist bevorzugt in einer Gesamtmenge von 0,2 bis 3 Gew.-%, besonders bevorzugt 0,5 - 2 Gew.-%, außerordentlich bevorzugt 0,8 bis 1,5 Gew.-%, jeweils bezogen auf das Gewicht des erfindungsgemäßen Mittels, enthalten.

Mindestens eine Polymer oder Copolymer, ausgewählt aus vernetzten Homopolymeren von Acrylsäure, vernetzten Homopolymeren von 2-Methyl-2[(1-oxo-2-propenyl)amino]-1-propansulfon-säure, vernetzten Copolymeren aus Methacrylsäure und C1-C4-Alkylacrylaten, vernetzten Copolymeren aus Acrylsäure und C1-C4-Alkylmethacrylaten, vernetzten Copolymeren aus 2-Methyl-2[(1-oxo-2-propenyl)amino]-1-propansulfonsäure und Acrylamid, vernetzten Copolymeren aus 2-Methyl-2[(1-oxo-2-propenyl)amino]-1-propansulfonsäure und Vinylpyrrolidon, vernetzten Copolymeren aus 2-Methyl-2[(1-oxo-2-propenyl)amino]-1-propansulfonsäure und 2-Hydroxyethylacrylat, vernetzten Copolymeren aus 2-Methyl-2[(1-oxo-2-propenyl)amino]-1-propansulfonsäure und Acrylsäure, vernetzten Copolymeren aus Acrylsäure und C10-30-Alkylacrylaten, vernetzten Terpolymeren und Quaterpolymeren der 2-Methyl-2[(1-oxo-2-propenyl)amino]-1-propansulfonsäure oder der Acrylsäure oder der Methacrylsäure sowie Mischungen dieser Polymere, ist bevorzugt in einer Gesamtmenge von 0,2 bis 3 Gew.-%, besonders bevorzugt 0,5 - 2 Gew.-%, außerordentlich bevorzugt 0,8 bis 1,5 Gew.-%, jeweils bezogen auf das Gewicht des erfindungsgemäßen Mittels, enthalten, wobei zwingend mindestens ein vernetztes Acrylsäure-Homopolymer enthalten ist.

Weitere besonders bevorzugte erfindungsgemäße Mittel sind dadurch gekennzeichnet, dass 0,2 bis 3 Gew.-%, besonders bevorzugt 0,5 - 2 Gew.-%, außerordentlich bevorzugt 0,8 bis 1,5 Gew.-% Xanthan und mindestens ein vernetztes Acrylsäure-Homopolymer in einer Gesamtmenge von 0,2 bis 3 Gew.-%, besonders bevorzugt 0,5 - 2 Gew.-%, außerordentlich bevorzugt 0,8 bis 1,5 Gew.-%, jeweils bezogen auf das Gewicht des erfindungsgemäßen Mittels, enthalten sind.

Als dritte erfindungswesentliche Komponente enthalten die erfindungsgemäßen Mittel mindestens ein Alkalisierungsmittel. Bevorzugt ist das Alkalisierungsmittel ausgewählt aus Ammoniak und mindestens einem Alkanolamin. Erfindungsgemäß bevorzugte Alkanolamine sind ausgewählt aus Alkanolaminen aus primären, sekundären oder tertiären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methyl-propan-2-ol, 3-Aminopropan-1,2-diol, 2-Amino-2-methylpropan-1,3-diol, 2-Amino-2-ethyl-1,3-propandiol, N,N-Dimethyl-ethanolamin, Triethanolamin, Diethanolamin und Triisopropanolamin. Erfindungsgemäß ganz besonders bevorzugte Alkanolamine sind ausgewählt aus der Gruppe 2-Aminoethan-1-ol (Monoethanolamin), 2-Amino-2-methylpropan-1-ol, 2-Amino-2-methyl-propan-1,3-diol und Triethanolamin. Besonders bevorzugte Mittel enthalten Monoethanolamin als Alkanolamin. Bevorzugt ist das mindestens eine Alkanolamin in einer Gesamtmenge von 0,05 bis 10 Gew.-%, insbesondere von 0,5 bis 6 Gew.-%, besonders bevorzugt 1 - 3 Gew.-%, jeweils bezogen auf das Gewicht des erfindungsgemäßen Mittels, enthalten.

Weitere erfindungsgemäß bevorzugte Alkalisierungsmittel sind ausgewählt aus anorganischen Alkalisierungsmitteln. Ammoniak, ebenfalls ein anorganisches Alkalisierungsmittel, ist allerdings weniger bevorzugt, da es sich bei der Anwendung zu früh verflüchtigen kann. Bevorzugte erfindungsgemäße Mittel und Anwendungsmischungen enthalten daher, jeweils bezogen auf ihr Gewicht, 0 bis 0,5 Gew.-%, bevorzugt null Gew.-% Ammoniak.

Bevorzugte anorganische Alkalisierungsmittel sind ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat sowie Mischungen hiervon. Weitere erfindungsgemäß bevorzugte Alkalisierungsmittel sind ausgewählt aus basischen Aminosäuren, besonders bevorzugt ausgewählt aus der Gruppe, die gebildet wird aus L-Arginin, D-Arginin, D/L-Arginin, L-Lysin, D-Lysin, D/L-Lysin sowie Mischungen hiervon. Erfindungsgemäß besonders bevorzugte basische Aminosäuren sind ausgewählt aus L-Arginin, D-Arginin und D/L-Arginin. Bevorzugte erfindungsgemäße Mittel enthalten mindestens ein von Alkanolaminen und Ammoniak verschiedenes Alkalisierungsmittel in einer Gesamtmenge von 0,05 bis 5 Gew.-%, insbesondere von 0,5 bis 3 Gew.-%, jeweils bezogen auf das Gewicht des Mittels. Bei der Ermittlung der optimalen Menge an Alkalisierungsmittel ist zu beachten, dass das erfindungsgemäße Mittel einen pH-Wert im Bereich von 7,0 bis 8,8, bevorzugt im Bereich von 7,5 bis 8,5, aufweist, jeweils gemessen bei 22°C.

Als vierte erfindungswesentliche Komponente enthalten die erfindungsgemäßen Mittel mindestens einen Lösevermittler, ausgewählt aus Fettalkoholglycolethern, Fettalkoholen und Fettsäureglycerinestern, die jeweils ethoxyliert und optional propoxyliert sind.

Unter Fettalkoholen werden bevorzugt C₈-C₂₄-Alkanole, die linear oder verzweigt und ungesättigt oder gesättigt sein können, verstanden. Typische Beispiele sind Caprylalkohol, Caprinalkohol, Undecylalkohol, Laurylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol und natürliche Fettalkohole, wie Kokosalkohol. Unter Fettsäuren werden bevorzugt C₈-C₂₄-Alkansäuren, die linear oder verzweigt und ungesättigt oder gesättigt sein können, verstanden. Typische Beispiele sind Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure und natürliche Fettsäuren, wie Kokosfettsäuren.

Erfindungsgemäß bevorzugte Mittel enthalten mindestens einen Lösevermittler, der ausgewählt ist aus ethoxylierten C₈-C₂₄-Alkanolen mit 9 - 100 Mol Ethylenoxid pro Mol, C₈-C₂₄-Alkylepoxid-Ethylenglycol-Anlagerungsprodukten, die mit 5 - 20 Mol Ethylenoxid pro Mol ethoxyliert und mit 1 Mol Propylenoxid pro Mol propoxyliert sind, mit 9 - 100 Mol Ethylenoxid pro Mol ethoxylierten Glycerinmono, -di- oder -triestern von linearen gesättigten und ungesättigten C₁₂ - C₃₀-Carbonsäuren, die hydroxyliert sein können, insbesondere diejenigen von Myristinsäure, Palmitinsäure, Stearinsäure, Ölsäure oder von Mischungen dieser Fettsäuren, ethoxylierten C₈-C₂₄-Carbonsäuren mit 9 - 100 Mol Ethylenoxid pro Mol, mit 9 - 100 Mol Ethylenoxid pro Mol ethoxylierten Fettsäureglycerinestern von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, mit 9 - 100 Mol Ethylenoxid pro Mol ethoxylierten Sorbitanmonoestern von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, sowie Mischungen der vorgenannten Substanzen.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die Anzahl der Ethylenoxid-Einheiten pro Molekül, für Zahlen von 9 - 100, vorzugsweise 9 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 9 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet.

Die ethoxylierten C₈-C₂₄-Carbonsäuren haben die Formel R²O(CH₂CH₂O)ₙH, wobei R²O steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 9 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie deren technische Mischungen. Auch Addukte von 9 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Besonders bevorzugt eingesetzt werden die C₁₂-C₁₈-Alkanole mit jeweils 9 - 30 Einheiten Ethylenoxid pro Molekül sowie Mischungen dieser Substanzen, insbesondere Ceteth-10, Ceteth-12, Ceteth-20, Ceteth-30, Steareth-10, Steareth-12, Steareth-20, Steareth-30, Ceteareth-10, Ceteareth-12, Ceteareth-20, Ceteareth-30, Laureth-12 und Beheneth-20.

Bevorzugte mit 20 - 100 Mol Ethylenoxid pro Mol ethoxylierte Fettsäureglycerinester von linearen gesättigten und ungesättigten C₁₂ - C₃₀-Carbonsäuren, die hydroxyliert sein können, sind ausgewählt aus PEG-40 Hydrogenated Castor Oil, PEG-60 Hydrogenated Castor Oil, PEG-80 Hydrogenated Castor Oil, PEG-40 Castor Oil, PEG-60 Castor Oil und PEG-80 Castor Oil sowie Mischungen hiervon.

Bevorzugte mit 20 - 100 Mol Ethylenoxid pro Mol ethoxylierte Sorbitanmonoester von linearen gesättigten und ungesättigten C₁₂ - C₃₀-Carbonsäuren, die hydroxyliert sein können, sind ausgewählt aus Polysorbate-20, Polysorbate-40, Polysorbate-60 und Polysorbate-80.

Besonders bevorzugte Lösevermittler sind ausgewählt aus Fettalkoholglycolethern, die ethoxyliert und optional propoxyliert sind, insbesondere aus den Reaktionsprodukten von Ethylenglycol mit den Epoxiden von Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol, die mit 1-3 Einheiten Propylenoxid pro Molekül und 9 - 30 Einheiten Ethylenoxid pro Molekül alkoxyliert sind.

Ein außerordentlich bevorzugter Lösevermittler, ausgewählt aus Fettalkoholglycolethern, die jeweils ethoxyliert und optional propoxyliert sind, ist PPG-1-PEG-9 Lauryl Glycol Ether. Mit diesem Lösevermittler gelingt es vorzüglich, die Oxidationsfarbstoffvorprodukte so weit zu solubilisieren, dass ein möglichst transparentes Gel erhalten wird. Die Struktur von PPG-1-PEG-9 Lauryl Glycol Ether ist nachstehend skizziert. Die Indices a und c stehen bevorzugt für den Wert 1, die Indices b und d stehen bevorzugt für den Wert 9. Ein bevorzugter PPG-1-PEG-9 Lauryl Glycol Ether ist das Handelsprodukt Eumulgin L von BASF.

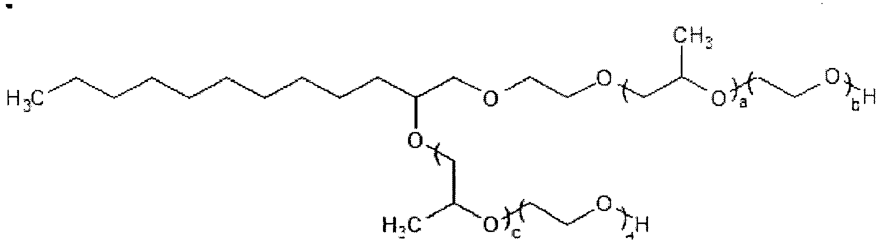

Bevorzugte erfindungsgemäße Mittel enthalten mindestens einen Lösevermittler, ausgewählt aus Fettalkoholglycolethern, Fettalkoholen und Fettsäureglycerinestern, die jeweils ethoxyliert und optional propoxyliert sind, in einer Gesamtmenge von 0,1 bis 10 Gew.-%, insbesondere in einer Gesamtmenge von 0,5 bis 5 Gew.-%, weiter bevorzugt in einer Gesamtmenge von 1 bis 4 Gew.-%, außerordentlich bevorzugt in einer Gesamtmenge von 2 bis 3 Gew.-%, jeweils bezogen auf das Gewicht des Mittels.

Weitere bevorzugte erfindungsgemäße Mittel enthalten mindestens einen Lösevermittler, ausgewählt aus ethoxylierten C₈-C₂₄-Alkanolen mit 9 - 100 Mol Ethylenoxid pro Mol, C₈-C₂₄-Alkylepoxid-Ethylenglycol-Anlagerungsprodukten, die mit 5 - 20 Mol Ethylenoxid pro Mol ethoxyliert und mit 1 Mol Propylenoxid pro Mol propoxyliert sind, mit 9 - 100 Mol Ethylenoxid pro Mol ethoxylierten Glycerinmono, -di- oder -triestern von linearen gesättigten und ungesättigten C₁₂ - C₃₀-Carbonsäuren, die hydroxyliert sein können, insbesondere diejenigen von Myristinsäure, Palmitinsäure, Stearinsäure, Ölsäure oder von Mischungen dieser Fettsäuren, ethoxylierten C₈-C₂₄-Carbonsäuren mit 9 - 100 Mol Ethylenoxid pro Mol, mit 9 - 100 Mol Ethylenoxid pro Mol ethoxylierten Fettsäureglycerinestern von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, mit 9 - 100 Mol Ethylenoxid pro Mol ethoxylierten Sorbitanmonoestern von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, sowie Mischungen der vorgenannten Substanzen, in einer Gesamtmenge von 0,1 bis 10 Gew.-%, insbesondere in einer Gesamtmenge von 0,5 bis 5 Gew.-%, weiter bevorzugt in einer Gesamtmenge von 1 bis 4 Gew.-%, außerordentlich bevorzugt in einer Gesamtmenge von 2 bis 3 Gew.-%, jeweils bezogen auf das Gewicht des Mittels.

Ein außerordentlich bevorzugtes erfindungsgemäßes Mittel enthält 0,1 bis 10 Gew.-%, insbesondere 0,5 bis 5 Gew.-%, weiter bevorzugt 1 bis 4 Gew.-%, außerordentlich bevorzugt 2 bis 3 Gew.-%, PPG-1-PEG-9 Lauryl Glycol Ether, jeweils bezogen auf das Gewicht des Mittels.

Wie vorstehend gesagt, sind bevorzugte erfindungsgemäße Mittel möglichst transparent.

Erfindungsgemäße Mittel sind daher gekennzeichnet durch eine Trübung im Bereich von null bis 80 NTU (Nephelometric Turbidity Unit), bevorzugt von maximal 60 NTU, besonders bevorzugt von maximal 50 NTU, außerordentlich bevorzugt von maximal 40 NTU, jeweils gemessen bei 22°C gemäß DIN EN ISO 7027-C2.

Sofern die erfindungsgemäßen Mittel Mittel zum Permanentfärben und ggf. gleichzeitigen Bleichen keratinischer Fasern sind, enthalten sie mindestens ein Oxidationsfarbstoffvorprodukt. Vorzugsweise enthält das Mittel eine oder mehrere Entwicklerkomponenten sowie gegebenenfalls eine oder mehrere Kupplerkomponenten.

Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 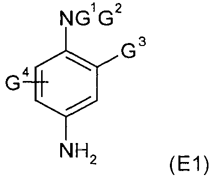 wobei
- G¹ steht für ein Wasserstoffatom, einen C₁-C₄-Alkylrest, einen C₁-C₄)-Monohydroxyalkylrest, einen C₂-C₄-Polyhydroxyalkylrest, einen C₁-C₄-Alkoxy-(C₁-C₄)-alkylrest, einen 4-Aminophenylrest oder einen C₁-C₄-Alkylrest, der mit einer stickstoffhaltigen Gruppe, einem Phenyl- oder einem 4-Aminophenylrest substituiert ist;
- G² steht für ein Wasserstoffatom, einen C₁-C₄-Alkylrest, einen C₁-C₄-Monohydroxyalkylrest, einen C₂-C₄-Polyhydroxyalkylrest, einen C₁-C₄-Alkoxy-(C₁-C₄)-alkylrest oder einen C₁-C₄-Alkylrest, der mit einer stickstoffhaltigen Gruppe substituiert ist;
- G³ steht für ein Wasserstoffatom, ein Halogenatom, wie ein Chlor-, Brom-, Iod- oder Fluoratom, einen C₁-C₄-Alkylrest, einen C₁-C₄-Monohydroxyalkylrest, einen C₂-C₄-Polyhydroxyalkylrest, einen C₁-C₄-Hydroxyalkoxyrest, einen C₁-C₄-Alkoxy-(C₁-C₄)-alkylrest, einen C₁-C₄-Acetylami-noalkoxyrest, einen Mesylamino-(C₁-C₄)-alkoxyrest oder einen C₁-C₄-Carbamoylaminoalkoxyrest;
- G⁴ steht für ein Wasserstoffatom, ein Halogenatom, einen C₁-C₄-Alkylrest oder einen C₁-C₄-Alkoxy-(C₁-C₄)-alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Besonders bevorzugte p-Phenylendiamine der Formel (E1) werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(2-hydroxyethyl)-p-phenylendiamin, 4-N,N-Bis-(2-hydroxyethyl)-amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N,N-(Ethyl,2-hydroxyethyl)-p-phenylendiamin, N-(2,3-Dihydroxypropyl)-p-phenylendiamin, N-(4-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen. Erfindungsgemäß ganz besonders bevorzugte p-Phenylendiaminderivate der Formel (E1) sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, 2-Methoxymethyl-p-phenylendiamin sowie deren physiologisch verträglichen Salze.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze, 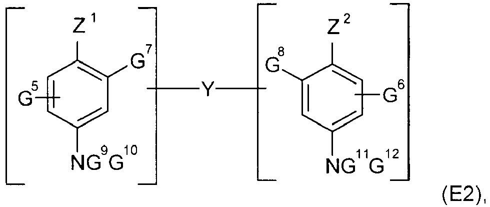 wobei
- Z¹ und Z² stehen unabhängig voneinander für einen Hydroxyl- oder NH₂-Rest, der gegebenenfalls durch einen C₁-C₄-Alkylrest, durch einen C₁-C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁-C₈-Alkoxyreste substituiert sein kann, oder eine direkte Bindung,
- G⁵ und G⁶ stehen unabhängig voneinander für ein Wasserstoff- oder Halogenatom, einen C₁-C₄-Alkylrest, einen C₁-C₄-Monohydroxyalkylrest, einen C₂-C₄-Polyhydroxyalkylrest, einen C₁-C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁-C₄-Alkylrest,
mit der Maßgabe, dass die Verbindungen der Formel (E2) nur eine Verbrückung Y pro Molekül enthalten.

Bevorzugte zweikernige Entwicklerkomponenten der Formel (E2) werden insbesondere aus mindestens einer der folgenden Verbindungen ausgewählt: N,N'-Bis-(2-hydroxyethyl)-N,N'-bis-(4-aminophenyl)-1,3-diamino-propan-2-ol, N,N'-Bis-(2-hydroxyethyl)-N,N'-bis-(4-aminophenyl)ethylendiamin, N,N'-Bis-(4-aminophenyl)tetramethylendiamin, N,N'-Bis-(2-hydroxyethyl)-N,N'-bis-(4-aminophenyl)-tetramethylendiamin, N,N'-Bis-(4-(methylamino)phenyl)tetramethylendiamin, N,N'-Diethyl-N,N'-bis-(4-amino-3-methylphenyl)ethylendiamin, Bis-(2-hydroxy-5-aminophenyl)methan, N,N'-Bis-(4-aminophenyl)-1,4-diazacycloheptan, N,N'-Bis-(2-hydroxy-5-aminobenzyl)piperazin, N-(4-Aminophenyl)-p-phenylendiamin und 1,10-Bis-(2',5'-diaminophenyl)-1,4,7,10-tetraoxadecan sowie ihre physiologisch verträglichen Salze. Ganz besonders bevorzugte zweikernige Entwicklerkomponenten der Formel (E2) werden ausgewählt unter N,N'-Bis-(2-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines deren physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3), 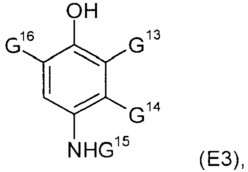 wobei
- G¹³ steht für ein Wasserstoffatom, ein Halogenatom, einen C₁-C₄-Alkylrest, einen C₁-C₄-Monohydroxyalkylrest, einen C₂-C₄-Polyhydroxyalkylrest, einen C₁-C₄-Alkoxy-(C₁-C₄)-alkylrest, einen C₁-C₄-Aminoalkylrest, einen Hydroxy-(C₁-C₄)-alkylaminorest, einen C₁-C₄-Hydroxyalkoxyrest, einen C₁-C₄-Hydroxyalkyl-(C₁-C₄)-aminoalkylrest oder einen Di-[(C₁-C₄)-alkyl]amino-(C₁-C₄)-alkylrest, und
- G¹⁴ steht für ein Wasserstoff- oder Halogenatom, einen C₁-C₄-Alkylrest, einen C₁-C₄-Monohydroxyalkylrest, einen C₂-C₄-Polyhydroxyalkylrest, einen C₁-C₄-Alkoxy-(C₁-C₄)-alkylrest, einen C₁-C₄-Aminoalkylrest oder einen C₁-C₄-Cyanoalkylrest,
- G¹⁵ steht für Wasserstoff, einen C₁-C₄-Alkylrest, einen C₁-C₄-Monohydroxyalkylrest, einen C₂-C₄-Polyhydroxyalkylrest, einen Phenylrest oder einen Benzylrest, und
- G¹⁶ steht für Wasserstoff oder ein Halogenatom.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethylaminomethyl)phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)phenol sowie ihre physiologisch verträglichen Salze. Besonders bevorzugte Verbindungen der Formel (E3) sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol. Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol. Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen. Besonders bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Besonders bevorzugte Pyrazol-Derivate sind insbesondere die Verbindungen, die ausgewählt werden unter 4,5-Diamino-1-methylpyrazol, 4,5-Diamino-1-(β-hydroxyethyl)-pyrazol, 3,4-Diaminopyrazol, 4,5-Diamino-1-(4-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol sowie deren physiologisch verträglichen Salze. Bevorzugte Pyrazolopyrimidine sind Pyrazolo[1,5-a]pyrimidine. Unter den Pyrazolo-[1,5-a]pyrimidinen sind wiederum Pyrazolo[1,5-a]pyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethyl-pyrazolo[1,5-a]pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]pyrimidin-3-ylamino)-ethanol, 2-[(3-Aminopyrazolo[1,5-a]pyrimidin-7-yl)-(2-hydroxy-ethyl)-amino]-ethanol, 2-[(7-Aminopyrazolo[1,5-a]pyrimidin-3-yl)-(2-hydroxy-ethyl)-amino]-ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen, wenn ein tautomeres Gleichgewicht vorhanden ist, besonders bevorzugt.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin sowie deren physiologisch verträglichen Salzen.

Bevorzugte erfindungsgemäße Mittel enthalten mindestens eine Entwicklerkomponente in einer Gesamtmenge von 0,005 bis 5 Gew.-%, bevorzugt 0,1 bis 3 Gew.-%, besonders bevorzugt 0,5 bis 2 Gew.-%, jeweils bezogen auf das Gewicht des Mittels.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Diese Gruppen stehen durch ein Doppelbindungssystem in Konjugation.

Erfindungsgemäße Kupplerkomponenten werden bevorzugt ausgewählt den Klassen von m-Aminophenol und/oder dessen Derivaten, m-Diaminobenzol und/oder dessen Derivaten, o-Diaminobenzol und/oder dessen Derivaten, Naphthalinderivaten mit mindestens einer Hydroxygruppe, Dibeziehungsweise Trihydroxybenzol und/oder deren Derivaten, Pyridinderivaten, Pyrimidinderivaten, Monohydroxyindol-Derivaten und/oder Monoaminoindol-Derivaten, Monohydroxyindolin-Derivaten und/oder Monoaminoindolin-Derivaten, Pyrazolonderivaten, wie 1-Phenyl-3-methylpyrazol-5-on, Morpholinderivate, wie 6-Hydroxybenzomorpholin oder 6-Amino-benzomorpholin, Chinoxalinderivaten, wie 6-Methyl-1,2,3,4-tetrahydrochinoxalin, sowie Gemischen aus zwei oder mehreren Verbindungen aus einer oder mehrerer dieser Klassen.

Bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol und deren physiologisch verträglichen Salzen. Bevorzugte m-Diaminobenzol-Kupplerkomponenten werden ausgewählt aus mindestens einer Verbindung aus der Gruppe, die gebildet wird aus 2-(2,4-Diaminophenoxy)ethanol, 1,3-Bis-(2,4-diaminophenoxy)propan, 1-Methoxy-2-amino-4-(2'-hydroxyethylamino)benzol, 1,3-Bis-(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)-aminobenzol und deren physiologisch verträglichen Salzen. Bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und deren physiologisch verträglichen Salzen. Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol. Bevorzugte Pyridinderivate werden ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 2,6-Dihydroxy-3,4-dimethylpyridin, 3,4-Diaminopyridin, 2-(2-Methoxyethyl)amino-3-amino-6-methoxypyridin, 2-(4'-Methoxyphenyl)amino-3-aminopyridin und deren physiologisch verträglichen Salzen. Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin. Bevorzugte Indolderivate werden ausgewählt aus mindestens einer Verbindung der Gruppe, die gebildet wird aus 4-Hydroxyindol, 6-Hydroxyindol und 7-Hydroxyindol und deren physiologisch verträglichen Salzen. Bevorzugte Indolinderivate werden ausgewählt aus mindestens einer Verbindung der Gruppe, die gebildet wird aus 4-Hydroxyindolin, 6-Hydroxyindolin und 7-Hydroxyindolin und deren physiologisch verträglichen Salzen. Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Erfindungsgemäß besonders bevorzugte Kupplerkomponenten sind ausgewählt unter m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2-Hydroxyethyl)amino-2-methylphenol, 3-(Diethylamino)phenol, N-Cyclopentyl-3-aminophenol, 1,3-Dihydroxy-5-(methylamino)benzol, 3-Ethylamino-4-methylphenol, 2,4-Dichlor-3-aminophenol, 2-(2,4-Diaminophenoxy)ethanol, 1,3-Bis-(2,4-diaminophenoxy)propan, 1-Methoxy-2-amino-4-(2-hydroxyethylamino)benzol, 1,3-Bis-(2,4-diaminophenyl)propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)-amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, Resorcinmonomethylether, 2-Methylresorcin, 5-Methylresorcin, 2,5-Dimethylresorcin, 2-Chlorresorcin, 4-Chlorresorcin, Pyrogallol, 1,2,4-Trihydroxybenzol, 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 2,6-Dihydroxy-3,4-dimethylpyridin, 3,4-Diaminopyridin, 2-(2-Methoxyethyl)amino-3-amino-6-methoxypyridin, 2-(4'-Methoxyphenyl)amino-3-aminopyridin, 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin, 2,3-Dihydroxynaphthalin, 4-Hydroxyindol, 6-Hydroxyindol, 7-Hydroxyindol, 4-Hydroxyindolin, 6-Hydroxyindolin, 7-Hydroxyindolin, 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin oder Gemischen dieser Verbindungen oder ihrer physiologisch verträglichen Salze.

Die Kupplerkomponenten sind bevorzugt in einer Gesamtmenge von 0,005 bis 20 Gew.-%, vorzugsweise 0,1 bis 5 Gew.-%, besonders bevorzugt 0,8 bis 3 Gew.-%, jeweils bezogen auf das erfindungsgemäße Mittel, enthalten.

Dabei werden Entwicklerkomponenten und Kupplerkomponenten im Allgemeinen in etwa äquimolaren Mengen zueinander eingesetzt. Wenn sich auch der äquimolare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Molverhältnis von 1:0,5 bis 1:3, insbesondere 1:1 bis 1:2, stehen können.

Zur weiteren Nuancierung der resultierenden Farbtöne kann es erfindungsgemäß bevorzugt sein, dem Mittel weiterhin mindestens einen direktziehenden Farbstoff zuzusetzen. Hierbei handelt es sich um Farbstoffmoleküle, die direkt auf das Substrat aufziehen und keinen oxidativen Prozeß zur Ausbildung der Farbe benötigen. Zu diesen Farbstoffen gehört beispielsweise das bereits aus dem Altertum zur Färbung von Körper und Haaren bekannte Henna. Heutzutage sind direktziehende Farbstoffe üblicherweise Nitrophenylendiamine, Nitroaminophenole, Azofarbstoffe, Anthrachinone oder Indophenole.

Die direktziehenden Farbstoffe werden jeweils bevorzugt in einer Menge von 0,001 bis 10 Gew.-%, jeweils bezogen auf das erfindungsgemäße Mittel, eingesetzt. Die Gesamtmenge an direktziehenden Farbstoffen beträgt vorzugsweise höchstens 20 Gew.-%. Direktziehende Farbstoffe können in anionische, kationische und nichtionische direktziehende Farbstoffe unterteilt werden.

Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 sowie Tetrabromphenolblau und Bromphenolblau bekannten Verbindungen.

Bevorzugte kationische direktziehende Farbstoffe sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden. Die Verbindungen, die auch unter den Bezeichnungen Basic Yellow 87, Basic Orange 31 und Basic Red 51 bekannt sind, sind ganz besonders bevorzugte kationische direktziehende Farbstoffe.

Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.

Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)-amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu und Alkannawurzel enthalten sind.

Ein weiterer Gegenstand der vorliegenden Anmeldung ist ein anwendungsbereites Mittel (auch als Anwendungsmischung bezeichnet) zum Bleichen und/oder Färben von keratinischen Fasern, bestehend aus einer Mischung aus

25 - 75 Gew.-%, bevorzugt 40 bis 60 Gew.-%, besonders bevorzugt 50 Gew.-%, jeweils bezogen auf das Gewicht der Anwendungsmischung, eines erfindungsgemäßen oder erfindungsgemäß bevorzugten Mittels mit einem pH-Wert im Bereich von 7,0 bis 8,8, gemessen bei 22°C, enthaltend
- 80 - 93 Gew.-%, bezogen auf das Mittel, Wasser oder einer Mischung aus Wasser und mindestens einem wasserlöslichen Polyol, wobei mindestens 30 Gew.-% Wasser, bezogen auf das Mittel, enthalten sind, weiterhin
- als Hydrogel bildendes Polymere Xanthan und mindestens ein vernetztes Acrylsäure-Homopolymer weiterhin
- mindestens ein Alkalisierungsmittel, weiterhin
- mindestens einen Lösevermittler, ausgewählt aus Fettalkoholglycolethern, Fettalkoholen, Fettsäureglycerinestern und Fettsäuresorbitanestern, die jeweils ethoxyliert und optional propoxyliert sind,
- optional mindestens ein Oxidationsfarbstoffvorprodukt und/oder mindestens einen direktziehenden Farbstoff,
- wobei das Mittel als Hydrogel vorliegt und
- gekennzeichnet ist durch eine Trübung im Bereich von null bis 80 NTU, bevorzugt von maximal 60 NTU, besonders bevorzugt von maximal 50 NTU, außerordentlich bevorzugt von maximal 40 NTU, jeweils gemessen bei 22°C gemäß DIN EN ISO 7027-C2,
und
75 - 25 Gew.-%, bevorzugt 60 bis 40 Gew.-%, besonders bevorzugt 50 Gew.-%, jeweils bezogen auf das Gewicht der Anwendungsmischung, einer Oxidationsmittelzubereitung, enthaltend
- 75 - 95 Gew.-%, bezogen auf das Gewicht der Oxidationsmittelzubereitung, Wasser oder einer Mischung aus Wasser und mindestens einem wasserlöslichen Polyol, bevorzugt ausgewählt aus mindestens einem wasserlöslichen mehrwertigen C₂ - C₉-Alkanol mit 2 - 6 Hydroxylgruppen und/oder mindestens einem wasserlöslichen Polyethylenglycol mit 3 - 20 Ethylenoxid-Einheiten sowie Mischungen hiervon, besonders bevorzugt ausgewählt aus 1,2-Propylenglycol, 1,3-Propylenglycol, 2-Methyl-1,3-propandiol, Glycerin, 1,2-Butylenglycol, 1,3-Butylenglycol, 1,4-Butylenglycol, 1,2-Pentandiol, 1,5-Pentandiol, 1,2-Hexandiol, 1,6-Hexandiol, 2-Methyl-2,4-pentandiol, 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebigen Isomeren-Gemischen von cis- und trans-1,4-Dimethylolcyclohexan, PEG-3, PEG-4, PEG-6, PEG-7, PEG-8, PEG-9, PEG-10, PEG-12, PEG-14, PEG-16, PEG-18 und PEG-20 sowie Mischungen hiervon, weiterhin
- mindestens ein Hydrogel bildendes Polymer, ausgewählt aus der Gruppe, gebildet aus Polysacchariden, Polymeren und Copolymeren der Acrylsäure, Polymeren und Copolymeren der Methacrylsäure und Polymeren und Copolymeren der 2-Methyl-2[(1-oxo-2-propenyl)amino]-1-propansulfonsäure, sowie aus Mischungen hiervon, weiterhin
- 1,0 bis 23,0 Gew.-% Wasserstoffperoxid, bezogen auf das Gewicht der Oxidationsmittelzubereitung,
- wobei die Oxidationsmittelzubereitung einen pH-Wert, jeweils gemessen bei 22°C, im Bereich von 2,0 bis 6,5, bevorzugt im Bereich von 3,0 bis 4,5 aufweist.

Bezüglich weiterer bevorzugter Ausführungsformen des erfindungsgemäßen anwendungsbereiten Mittels (vorstehend auch als Anwendungsmischung bezeichnet) gilt mutatis mutandis das zu den erfindungsgemaßen Mitteln Gesagte.

Die Ausbildung der Farbstoffe in oxidativen Färbemitteln erfolgt erst durch den Einfluss eines Oxidationsmittels, üblicherweise wird hierfür Wasserstoffperoxid verwendet. In einer bevorzugten Ausführungsform wird das Wasserstoffperoxid als wässrige Lösung verwendet. Erfindungsgemäß bevorzugte Oxidationsmittelzubereitungen sind dadurch gekennzeichnet, dass sie 1,0 bis 23,0 Gew.-%, weiter bevorzugt 2,5 bis 21,0 Gew.-%, besonders bevorzugt 4,0 bis 20,0 Gew.-% und ganz besonders bevorzugt 5,0 bis 18,0 Gew.-% Wasserstoffperoxid (berechnet als 100 %-iges H₂O₂) enthalten, jeweils bezogen auf das Gewicht der Oxidationsmittelzubereitung.

Es hat sich als vorteilhaft erwiesen, wenn die erfindungsgemäßen Oxidationsmittelzubereitungen zur Stabilisierung des Wasserstoffperoxids zusätzlich mindestens einen Stabilisator oder Komplexbildner enthalten. Besonders bevorzugte Stabilisatoren sind insbesondere EDTA, EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Das mindestens eine wasserlösliche Polyol ist bevorzugt in einer Gesamtmenge von 0,5 - 63 Gew.-%, besonders bevorzugt in einer Gesamtmenge von 1 - 50 Gew.-%, weiter besonders bevorzugt in einer Gesamtmenge von 2 - 30 Gew.-%, außerordentlich bevorzugt in einer Gesamtmenge von 4 - 10 Gew.-%, jeweils bezogen auf das Gewicht der Oxidationsmittelzubereitung, enthalten. Das mindestens eine Hydrogel bildende Polymer, ausgewählt aus der Gruppe, gebildet aus Polysacchariden, Polymeren und Copolymeren der Acrylsäure, Polymeren und Copolymeren der Methacrylsäure und Polymeren und Copolymeren der 2-Methyl-2[(1-oxo-2-propenyl)amino]-1-propansulfonsäure, sowie aus Mischungen hiervon, ist bevorzugt in gleicher Art und Menge enthalten, wie vorstehend für das erfindungsgemäße Mittel beschrieben.

Ganz besonders bevorzugt ist es, wenn das anwendungsbereite Mittel einen pH-Wert im Bereich von 6,0 bis 7,3, bevorzugt 6,2 bis 6,9, jeweils gemessen bei 22°C, aufweist. Dieser für ein oxidatives Haarfärbemittel sehr niedrige pH-Wert trägt deutlich zur Verringerung der Haarschädigung bei. Es kann bevorzugt sein, dass das erfindungsgemäße Mittel und/oder die Oxidationsmittelzubereitung mindestens eine Säure/Base-Puffersubstanz enthalten. Bevorzugte Puffersubstanzen sind die Phosphate, Hydrogenphosphate und Dihydrogenphosphate von Natrium und Kalium. Ganz besonders bevorzugt ist es, wenn das erfindungsgemäße Mittel Natriumphosphat, Natriumhydrogenphosphat, Natriumdihydrogenphosphat, Kaliumphosphat, Kaliumhydrogenphosphat und/oder Kaliumdihydrogenphosphat in einer Gesamtmenge von 0,1 bis 1,0 Gew.-%, bevorzugt 0,4 bis 0,8 Gew.-%, jeweils bezogen auf das Gewicht des erfindungsgemäßen Mittels, enthält.

Ein weiterer Gegenstand der vorliegenden Anmeldung ist ein Kit zum Färben und/oder Aufhellen von keratinischen Fasern, umfassend ein Mittel nach einem der Ansprüche 1 bis 6 44 und eine Oxidationsmittelzubereitung, enthaltend
- 75 - 93 Gew.-%, bezogen auf das Gewicht der Oxidationsmittelzubereitung, Wasser oder einer Mischung aus Wasser und mindestens einem wasserlöslichen Polyol, bevorzugt ausgewählt aus mindestens einem wasserlöslichen mehrwertigen C₂ - C₉-Alkanol mit 2 - 6 Hydroxylgruppen und/oder mindestens einem wasserlöslichen Polyethylenglycol mit 3 - 20 Ethylenoxid-Einheiten sowie Mischungen hiervon, besonders bevorzugt ausgewählt aus 1,2-Propylenglycol, 1,3-Propylenglycol, 2-Methyl-1,3-propandiol, Glycerin, 1,2-Butylenglycol, 1,3-Butylenglycol, 1,4-Butylenglycol, 1,2-Pentandiol, 1,5-Pentandiol, 1,2-Hexandiol, 1,6-Hexandiol, 2-Methyl-2,4-pentandiol, 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebigen Isomeren-Gemischen von cis- und trans-1,4-Dimethylolcyclohexan, PEG-3, PEG-4, PEG-6, PEG-7, PEG-8, PEG-9, PEG-10, PEG-12, PEG-14, PEG-16, PEG-18 und PEG-20 sowie Mischungen hiervon, weiterhin
- mindestens ein Hydrogel bildendes Polymer, ausgewählt aus der Gruppe, gebildet aus Polysacchariden, Polymeren und Copolymeren der Acrylsäure, Polymeren und Copolymeren der Methacrylsäure und Polymeren und Copolymeren der 2-Methyl-2[(1-oxo-2-propenyl)amino]-1-propansulfonsäure, sowie aus Mischungen hiervon, weiterhin
- 1,0 bis 23 Gew.-% Wasserstoffperoxid, bezogen auf das Gewicht der Oxidationsmittelzubereitung,
- wobei die Oxidationsmittelzubereitung einen pH-Wert, jeweils gemessen bei 22°C, im Bereich von 2,0 bis 6,5, bevorzugt im Bereich von 3,0 bis 4,5 aufweist.

Bezüglich weiterer bevorzugter Ausführungsformen des erfindungsgemäßen Kits gilt mutatis mutandis das zu den erfindungsgemäßen Mitteln, das zu den erfindungsgemäßen anwendungsbereiten Mitteln (also den erfindungsgemäßen Anwendungsmischungen) und das zu den erfindungsgemäß verwendeten Oxidationsmittelzubereitungen Gesagte.

Ein weiterer Gegenstand der vorliegenden Anmeldung ist ein Verfahren zum Färben und/oder Aufhellen von keratinischen Fasern, das folgende Verfahrensschritte umfasst:
- optional Aufbringen eines Vorbehandlungsmittels M1 auf die Fasern, dann
- Herstellen und Aufbringen eines anwendungsbereiten Mittels M2 gemäß Anspruch 7 oder 8 auf die Fasern,
- Abspülen dieses Mittels M2 nach einer Zeit von 1 bis 60 Minuten von der Faser
- und anschließend gegebenenfalls Auftragen eines Nachbehandlungsmittels M3 auf die Faser und Abspülen nach einer Einwirkzeit von 0,5 bis 30 Minuten.

Bezüglich weiterer bevorzugter Ausführungsformen des erfindungsgemäßen Verfahrens gilt mutatis mutandis das zu den erfindungsgemäßen Mitteln und das zu den erfindungsgemäß verwendeten Oxidationsmittelzubereitungen Gesagte.

### Beispiel

In der folgenden Tabelle ist ein erfindungsgemäßes Färbegel dargestellt, das einen pH-Wert (22°C) von 8,2 aufweist. (alle Angaben in Gew.-%, bezogen auf das erfindungsgemäße Mittel)

| | |
|---|---|
| 1,2-Propylenglycol | 5,000000 |
| PPG-1-PEG-9 Lauryl Glycol Ether | 2,000000 |
| Toluene-2,5-Diamine Sulfate | 1,530000 |
| Xanthan Gum | 1,000000 |
| Ethanolamine | 0,995000 |
| Carbomer | 0,800000 |
| PEG-12 Dimethicone | 0,790000 |
| Potassium Phosphate | 0,500000 |
| Parfum (Fragrance) | 0,400000 |
| Sodium Methylparaben | 0,390000 |
| 2-Methylresorcinol | 0,340000 |
| 2-Amino-3-Hydroxypyridine | 0,312000 |
| 2-Amino-6-Chloro-4-Nitrophenol | 0,220000 |
| Citric Acid | 0,200000 |
| Niacinamide | 0,150000 |
| Panthenol | 0,150000 |
| Sodium Sulfite | 0,100000 |
| Ascorbic Acid | 0,100000 |
| Polyquaternium-7 | 0,090000 |
| 4-Amino-2-Hydroxytoluene | 0,072000 |
| PEG-14M | 0,070000 |
| m-Aminophenol | 0,069650 |
| Etidronic Acid | 0,060000 |
| 4-Chlororesorcinol | 0,017000 |
| Aqua (Water, Eau) | ad 100,000 |

In der folgenden Tabelle ist eine erfindungsgemäß verwendete Oxidationsmittelzubereitung dargestellt, die einen pH-Wert (22°C) von 4,0 aufweist (alle Angaben in Gew.-%, bezogen auf die erfindungsgemäß verwendete Oxidationsmittelzubereitung).

| | |
|---|---|
| Hydrogen Peroxide | 4,000000 |
| 1,2-Propylenglycol | 4,000000 |
| Xanthan Gum | 2,000000 |
| Etidronic Acid | 0,900000 |
| Sodium Hydroxide | 0,328500 |
| 2,6-Dicarboxypyridine | 0,100000 |
| Disodium Pyrophosphate | 0,030000 |
| Aqua (Water, Eau) | ad 100,000 |

Das erfindungsgemäße Färbegel wurde direkt vor der Anwendung mit der vorstehend dargestellten Oxidationsmittelzubereitung bei Raumtemperatur im Gewichtsverhältnis von 1:1 versetzt und innig vermischt. Das so erhaltene anwendungsbereite Mittel wies einen pH-Wert bei 22°C von 6,5 auf. Das anwendungsbereite Mittel wurde anschließend auf Haarsträhnen aufgetragen und nach einer Einwirkzeit von 30 Minuten wieder ausgespült.

Die Strähnen wurden getrocknet.

Auf dieselbe Weise wurde ein konventionelles anwendungsbereites Mittel mit einem pH-Wert von 10,5 getestet.

Ein Vergleich der Cysteinsäurewert zeigte, dass das nicht erfindungsgemäße anwendungsbereite Mittel mit einem pH-Wert von 10,5 einen deutlich höheren Cysteinsäurewert erzeugte als das erfindungsgemäße anwendungsbereite Mittel. Mit der Analyse der freien Cysteinsäure hat man ein Maß für die Haarschädidung (mehr Cysteinsäure ist gleichbedeutend mit größerer Haarschädigung).

## Patentansprüche

1. Mittel zum Färben und/oder Aufhellen von keratinischen Fasern, enthaltend:
- 80 - 93 Gew.-%, bezogen auf das Mittel, Wasser oder einer Mischung aus Wasser und mindestens einem wasserlöslichen Polyol, wobei mindestens 30 Gew.-% Wasser, bezogen auf das Mittel, enthalten sind, weiterhin
- als Hydrogel bildende Polymere Xanthan und mindestens ein vernetztes Acrylsäure-Homopolymer, weiterhin
- mindestens ein Alkalisierungsmittel, weiterhin
- mindestens einen Lösevermittler, ausgewählt aus Fettalkoholglycolethern, Fettalkoholen, Fettsäureglycerinestern und Fettsäuresorbitanestern, die jeweils ethoxyliert und optional propoxyliert sind,
- optional mindestens ein Oxidationsfarbstoffvorprodukt und/oder mindestens einen direktziehenden Farbstoff,
- wobei das Mittel einen pH-Wert im Bereich von 7,0 bis 8,8, gemessen bei 22°C, aufweist,
- als Hydrogel vorliegt und
- **gekennzeichnet ist durch** eine Trübung im Bereich von null bis 80 NTU, bevorzugt von maximal 60 NTU, besonders bevorzugt von maximal 50 NTU, außerordentlich bevorzugt von maximal 40 NTU, jeweils gemessen bei 22°C gemäß DIN EN ISO 7027-C2.

2. Mittel nach Anspruch 1, **dadurch gekennzeichnet, dass** keine anionischen Tenside, keine kationischen Tenside und keine Fettsäurediethanolamide enthalten sind.

3. Mittel nach Anspruch 1 oder 2, **dadurch gekennzeichnet, dass** das mindestens eine wasserlösliche Polyol ausgewählt ist aus mindestens einem wasserlöslichen mehrwertigen C₂ - C₉-Alkanol mit 2 - 6 Hydroxylgruppen und/oder mindestens einem wasserlöslichen Polyethylenglycol mit 3 -20 Ethylenoxid-Einheiten sowie Mischungen hiervon, bevorzugt ausgewählt aus 1,2-Propylenglycol, 1,3-Propylenglycol, 2-Methyl-1,3-propandiol, Glycerin, 1,2-Butylenglycol, 1,3-Butylenglycol, 1,4-Butylenglycol, 1,2-Pentandiol, 1,5-Pentandiol, 1,2-Hexandiol, 1,6-Hexandiol, 2-Methyl-2,4-pentandiol, 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebigen Isomeren-Gemischen von cis- und trans-1,4-Dimethylolcyclohexan, PEG-3, PEG-4, PEG-6, PEG-7, PEG-8, PEG-9, PEG-10, PEG-12, PEG-14, PEG-16, PEG-18 und PEG-20 sowie Mischungen hiervon.

4. Mittel nach einem der Ansprüche 1 bis 3, **dadurch gekennzeichnet, dass** das mindestens eine Alkalisierungsmittel ausgewählt ist aus Ammoniak und mindestens einem Alkanolamin, das bevorzugt ausgewählt ist aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methylpropanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Triethanolamin, Diethanolamin und Triisopropanolamin, sowie Mischungen der vorgenannten Substanzen.

5. Mittel nach einem der Ansprüche 1 bis 4, **dadurch gekennzeichnet, dass** der mindestens eine Lösevermittler ausgewählt ist aus ethoxylierten C₈-C₂₄-Alkanolen mit 9 - 100 Mol Ethylenoxid pro Mol, C₈-C₂₄-Alkylepoxid-Ethylenglycol-Anlagerungsprodukten, die mit 5 - 20 Mol Ethylenoxid pro Mol ethoxyliert und mit 1 Mol Propylenoxid pro Mol propoxyliert sind, mit 9 - 100 Mol Ethylenoxid pro Mol ethoxylierten Glycerinmono, -di- oder -triestern von linearen gesättigten und ungesättigten C₁₂ - C₃₀-Carbonsäuren, die hydroxyliert sein können, insbesondere diejenigen von Myristinsäure, Palmitinsäure, Stearinsäure, Ölsäure oder von Mischungen dieser Fettsäuren, ethoxylierten C₈-C₂₄-Carbonsäuren mit 9 - 100 Mol Ethylenoxid pro Mol, mit 9 - 100 Mol Ethylenoxid pro Mol ethoxylierten Sorbitanmonoestern von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, sowie Mischungen der vorgenannten Substanzen.

6. Mittel nach einem der Ansprüche 1 bis 5, **dadurch gekennzeichnet, dass** der mindestens eine Lösevermittler ausgewählt ist aus mindestens einer Verbindung mit der INCI-Bezeichnung PPG-1-PEG-9 Lauryl Glycol Ether.

7. Anwendungsbereites Mittel zum Bleichen und/oder Färben von keratinischen Fasern, bestehend aus einer Mischung aus
25 - 75 Gew.-%, bevorzugt 40 bis 60 Gew.-%, besonders bevorzugt 50 Gew.-% eines Mittels nach einem der Ansprüche 1 bis 6 und
75 - 25 Gew.-%, bevorzugt 60 bis 40 Gew.-%, besonders bevorzugt 50 Gew.-% einer Oxidationsmittelzubereitung, enthaltend
- 75 - 95 Gew.-%, bezogen auf das Gewicht der Oxidationsmittelzubereitung, Wasser oder einer Mischung aus Wasser und mindestens einem wasserlöslichen Polyol, bevorzugt ausgewählt aus mindestens einem wasserlöslichen mehrwertigen C₂ - C₉-Alkanol mit 2 - 6 Hydroxylgruppen und/oder mindestens einem wasserlöslichen Polyethylenglycol mit 3 - 20 Ethylenoxid-Einheiten sowie Mischungen hiervon, besonders bevorzugt ausgewählt aus 1,2-Propylenglycol, 1,3-Propylenglycol, 2-Methyl-1,3-propandiol, Glycerin, 1,2-Butylenglycol, 1,3-Butylenglycol, 1,4-Butylenglycol, 1,2-Pentandiol, 1,5-Pentandiol, 1,2-Hexandiol, 1,6-Hexandiol, 2-Methyl-2,4-pentandiol, 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebigen Isomeren-Gemischen von cis- und trans-1,4-Dimethylolcyclohexan, PEG-3, PEG-4, PEG-6, PEG-7, PEG-8, PEG-9, PEG-10, PEG-12, PEG-14, PEG-16, PEG-18 und PEG-20 sowie Mischungen hiervon, weiterhin
- mindestens ein Hydrogel bildendes Polymer, ausgewählt aus der Gruppe, gebildet aus Polysacchariden, Polymeren und Copolymeren der Acrylsäure, Polymeren und Copolymeren der Methacrylsäure und Polymeren und Copolymeren der2-Methyl-2[(1-oxo-2-propenyl)amino]-1-propansulfonsäure, sowie aus Mischungen hiervon, weiterhin
- 1,0 bis 23,0 Gew.-% Wasserstoffperoxid, bezogen auf das Gewicht der Oxidationsmittelzubereitung,
- wobei die Oxidationsmittelzubereitung einen pH-Wert, jeweils gemessen bei 22°C, im Bereich von 2,0 bis 6,5, bevorzugt im Bereich von 3,0 bis 4,5 aufweist.

8. Anwendungsbereites Mittel gemäß Anspruch 7, **gekennzeichnet durch** einen pH-Wert im Bereich von 6,0 bis 7,3, bevorzugt 6,2 bis 6,9, jeweils gemessen bei 22°C.

9. Kit zum Färben und/oder Aufhellen von keratinischen Fasern, umfassend ein Mittel nach einem der Ansprüche 1 bis 6 und eine Oxidationsmittelzubereitung, enthaltend
- 75 - 93 Gew.-%, bezogen auf das Gewicht der Oxidationsmittelzubereitung, Wasser oder einer Mischung aus Wasser und mindestens einem wasserlöslichen Polyol, bevorzugt ausgewählt aus mindestens einem wasserlöslichen mehrwertigen C₂ - C₉-Alkanol mit 2 - 6 Hydroxylgruppen und/oder mindestens einem wasserlöslichen Polyethylenglycol mit 3 - 20 Ethylenoxid-Einheiten sowie Mischungen hiervon, besonders bevorzugt ausgewählt aus 1,2-Propylenglycol, 1,3-Propylenglycol, 2-Methyl-1,3-propandiol, Glycerin, 1,2-Butylenglycol, 1,3-Butylenglycol, 1,4-Butylenglycol, 1,2-Pentandiol, 1,5-Pentandiol, 1,2-Hexandiol, 1,6-Hexandiol, 2-Methyl-2,4-pentandiol, 1,2,6-Hexantriol, 1,2-Octandiol, 1,8-Octandiol, Dipropylenglycol, Tripropylenglycol, Diglycerin, Triglycerin, Erythrit, Sorbit, cis-1,4-Dimethylolcyclohexan, trans-1,4-Dimethylolcyclohexan, beliebigen Isomeren-Gemischen von cis- und trans-1,4-Dimethylolcyclohexan, PEG-3, PEG-4, PEG-6, PEG-7, PEG-8, PEG-9, PEG-10, PEG-12, PEG-14, PEG-16, PEG-18 und PEG-20 sowie Mischungen hiervon, weiterhin
- mindestens ein Hydrogel bildendes Polymer, ausgewählt aus der Gruppe, gebildet aus Polysacchariden, Polymeren und Copolymeren der Acrylsäure, Polymeren und Copolymeren der Methacrylsäure und Polymeren und Copolymeren der 2-Methyl-2[(1-oxo-2-propenyl)amino]-1-propansulfonsäure, sowie aus Mischungen hiervon, weiterhin
- 1,0 bis 23 Gew.-% Wasserstoffperoxid, bezogen auf das Gewicht der Oxidationsmittelzubereitung,
- wobei die Oxidationsmittelzubereitung einen pH-Wert, jeweils gemessen bei 22°C, im Bereich von 2,0 bis 6,5, bevorzugt im Bereich von 3,0 bis 4,5 aufweist.

10. Verfahren zum Färben und/oder Aufhellen von keratinischen Fasern, **dadurch gekennzeichnet, dass**
- optional ein Vorbehandlungsmittel M1 auf die Fasern aufgebracht wird, dann
- ein anwendungsbereites Mittel M2 gemäß Anspruch 7 oder 8 hergestellt und auf die Fasern aufgebracht wird,
- dieses Mittel M2 nach einer Zeit von 1 bis 60 Minuten von der Faser abgespült wird
- und anschließend gegebenenfalls ein Nachbehandlungsmittel M3 auf die Faser aufgetragen und nach einer Einwirkzeit von 0,5 bis 30 Minuten wieder abgespült wird.

## Claims

1. An agent for dyeing and/or lightening keratin fibers, containing:
- 80-93 wt.%, based on the agent, water or a mixture of water and at least one water-soluble polyol, at least 30 wt.% water, based on the agent, being contained, additionally
- xanthan gum and at least one cross-linked acrylic acid homopolymer as hydrogel-forming polymers, additionally
- at least one alkalizing agent, additionally
- at least one solubilizer selected from fatty alcohol glycol ethers, fatty alcohols, fatty acid glycerol esters and fatty acid sorbitan esters, in each case ethoxylated and optionally propoxylated,
- optionally at least one oxidation dye precursor and/or at least one substantive dye,
- the agent having a pH in the range of from 7.0 to 8.8, measured at 22 °C,
- being present as a hydrogel, and
- being **characterized by** a turbidity in the range of from zero to 80 NTU, preferably no more than 60 NTU, particularly preferably no more than 50 NTU, exceptionally preferably no more than 40 NTU, in each case measured at 22 °C according to DIN EN ISO 7027-C2.

2. The agent according to claim 1, **characterized in that** no anionic surfactants, no cationic surfactants and no fatty acid diethanolamides are contained.

3. The agent according to claim 1 or 2, **characterized in that** the at least one water-soluble polyol is selected from at least one water-soluble polyvalent C₂-C₉ alkanol having 2-6 hydroxyl groups and/or at least one water-soluble polyethylene glycol having 3-20 ethylene oxide units and mixtures thereof, preferably selected from 1,2-propylene glycol, 1,3-propylene glycol, 2-methyl-1,3-propanediol, glycerol, 1,2-butylene glycol, 1,3-butylene glycol, 1,4-butylene glycol, 1,2-pentanediol, 1,5-pentanediol, 1,2-hexanediol, 1,6-hexanediol, 2-methyl-2,4-pentanediol, 1,2,6-hexanetriol, 1,2-octanediol, 1,8-octanediol, dipropylene glycol, tripropylene glycol, diglycerol, triglycerol, erythritol, sorbitol, cis-1,4-dimethylol cyclohexane, trans-1,4-dimethylol cyclohexane, any isomeric mixtures of cis- und trans-1,4-dimethylol cyclohexane, PEG-3, PEG-4, PEG-6, PEG-7, PEG-8, PEG-9, PEG-10, PEG-12, PEG-14, PEG-16, PEG-18 und PEG-20 and mixtures thereof.

4. The agent according to one of claims 1 to 3, **characterized in that** the at least one alkalizing agent is selected from ammonia and at least one alkanol amine, preferably selected from 2-aminoethane-1-ol (monoethanolamine), 3-aminopropane-1-ol, 4-aminobutane-1-ol, 5-aminopentane-1-ol, 1-aminopropane-2-ol (monoisopropanolamine), 1-amino-butane-2-ol, 1-aminopentane-2-ol, 1-aminopentane-3-ol, 1-aminopentane-4-ol, 2-amino-2-methylpropanol, 2-amino-2-methylbutanol, 3-amino-2-methylpropane-1-ol, 1-amino-2-methylpropane-2-ol, 3-aminopropane-1,2-diol, 2-amino-2-methylpropane-1,3-diol, 2-amino-2-ethyl-1,3-propanediol, N,N-dimethyl-ethanolamine, triethanolamine, diethanolamine und triisopropanolamine, and mixtures of the aforementioned substances.

5. The agent according to one of claims 1 to 4, **characterized in that** the at least one solubilizer is selected from ethoxylated C₈-C₂₄ alkanols having 9-100 mol ethylene oxide per mol, C₈-C₂₄ alkyl epoxide-ethyleneglycol addition products, ethoxylated using 5-20 mol ethylene oxide per mol and propoxylated using 1 mol propylene oxide per mol, glycerol monoesters, diesters or triesters, ethoxylated using 9-100 mol ethylene oxide per mol, of linear saturated and unsaturated C₁₂-C₃₀ carboxylic acids that can be hydroxylated, in particular those of myristic acid, palmitic acid, stearic acid, oleic acid or mixtures of said fatty acids, ethoxylated C₈-C₂₄ carboxylic acids having 9-100 mol ethylene oxide per mol, sorbitan monoesters, ethoxylated using 9-100 mol ethylene oxide per mol, of linear saturated and unsaturated C₁₂-C₃₀ carboxylic acids that can be hydroxylated, in particular those of myristic acid, palmitic acid, stearic acid or mixtures of said fatty acids, and mixtures of the aforementioned substances.

6. The agent according to one of claims 1 to 5, **characterized in that** the at least one solubilizer is selected from at least one compound having the INCI name PPG-1-PEG-9 lauryl glycol ether.

7. A ready-to-apply agent for bleaching and/or dyeing keratin fibers, consisting of a mixture of from 25 to 75 wt.%, preferably 40 to 60 wt.%, particularly preferably 50 wt.%, of an agent according to one of claims 1 to 6 and
from 75 to 25 wt.%, preferably 60 to 40 wt.%, particularly preferably 50 wt.%, of an oxidation agent preparation, containing
- from 75 to 95 wt.%, based on the weight of the oxidation agent preparation, water or a mixture of water and at least one water-soluble polyol, preferably selected from at least one water-soluble polyvalent C₂-C₉ alkanol having 2-6 hydroxyl groups and/or at least one water-soluble polyethylene glycol having 3-20 ethylene oxide units and mixtures thereof, particularly preferably selected from 1,2-propylene glycol, 1,3-propylene glycol, 2-methyl-1,3-propanediol, glycerol, 1,2-butyleneglycol, 1,3-butyleneglycol, 1,4-butyleneglycol, 1,2-pentanediol, 1,5-pentanediol, 1,2-hexanediol, 1,6-hexanediol, 2-methyl-2,4-pentanediol, 1,2,6-hexanetriol, 1,2-octanediol, 1,8-octanediol, dipropylene glycol, tripropylene glycol, diglycerol, triglycerol, erythritol, sorbitol, cis-1,4-dimethylol cyclohexane, trans-1,4-dimethylol cyclohexane, any isomeric mixtures of cis- and trans-1,4-dimethylol cyclohexane, PEG-3, PEG-4, PEG-6, PEG-7, PEG-8, PEG-9, PEG-10, PEG-12, PEG-14, PEG-16, PEG-18 and PEG-20 and mixtures thereof, additionally
- at least one hydrogel-forming polymer, selected from the group formed of polysaccharides, polymers and copolymers of acrylic acid, polymers and copolymers of methacrylic acid and polymers and copolymers of 2-methyl-2[(1-oxo-2-propenyl)amino]-1-propanesulfonic acid and mixtures thereof, additionally
- 1.0 to 23.0 wt.% hydrogen peroxide, based on the weight of the oxidation agent preparation,
- wherein the oxidation agent preparation has a pH in the range of from 2.0 to 6.5, preferably in the range of from 3.0 to 4.5, in each case measured at 22 °C.

8. The ready-to-apply agent according to claim 7, **characterized by** a pH in the range of from 6.0 to 7.3, preferably 6.2 to 6.9, in each case measured at 22 °C.

9. A kit for dyeing and/or lightening keratin fibers, comprising an agent according to one of claims 1 to 6 and an oxidation agent preparation, containing
- 75-93 wt.%, based on the weight of the oxidation agent preparation, water or a mixture of water and at least one water-soluble polyol, preferably selected from at least one water-soluble polyvalent C₂-C₉ alkanol having 2-6 hydroxyl groups and/or at least one water-soluble polyethylene glycol having 3-20 ethylene oxide units and mixtures thereof, particularly preferably selected from 1,2-propylene glycol, 1,3-propylene glycol, 2-methyl-1,3-propanediol, glycerol, 1,2-butylene glycol, 1,3-butylene glycol, 1,4-butylene glycol, 1,2-pentanediol, 1,5-pentanediol, 1,2-hexanediol, 1,6-hexanediol, 2-methyl-2,4-pentanediol, 1,2,6-hexanetriol, 1,2-octanediol, 1,8-octanediol, dipropylene glycol, tripropylene glycol, diglycerol, triglycerol, erythritol, sorbitol, cis-1,4-dimethylol cyclohexane, trans-1,4-dimethylol cyclohexane, any isomeric mixtures of cis- und trans-1,4-dimethylol cyclohexane, PEG-3, PEG-4, PEG-6, PEG-7, PEG-8, PEG-9, PEG-10, PEG-12, PEG-14, PEG-16, PEG-18 und PEG-20 and mixtures thereof
- at least one hydrogel-forming polymer, selected from the group formed of polysaccharides, polymers and copolymers of acrylic acid, polymers and copolymers of methacrylic acid and polymers and copolymers of 2-methyl-2[(1-oxo-2-propenyl)amino]-1-propanesulfonic acid and mixtures thereof, additionally
- 1.0 to 23 wt.% hydrogen peroxide, based on the weight of the oxidation agent preparation,
- wherein the oxidation agent preparation has a pH in the range of from 2.0 to 6.5, preferably in the range of from 3.0 to 4.5, in each case measured at 22 °C.

10. A method for dyeing and/or brightening keratin fibers, **characterized in that**
- a pre-treatment agent M1 is optionally applied to the fibers, then
- a ready-to-apply agent M2 according to claim 7 or 8 is prepared and applied to the fibers,
- said agent M2 is rinsed off the fiber after a period of 1-60 minutes,
- and subsequently, a post-treatment agent M3 is applied, if desired, to the fiber and is rinsed off again after a contact time of 0.5 to 30 minutes.

## Revendications

1. Agent de coloration et/ou d'éclaircissement des fibres kératiniques, comprenant :
- de 80 à 93 % en poids, par rapport à l'agent, d'eau ou d'un mélange d'eau et d'au moins un polyol soluble dans l'eau, l'agent contenant au moins 30 % en poids d'eau par rapport à l'agent, mais aussi
- en tant que polymères formant des hydrogels, le xanthane et au moins un homopolymère d'acide acrylique réticulé, mais aussi
- au moins un agent alcalinisant, mais aussi
- au moins un solubilisant choisi parmi les éthers de glycol d'alcool gras, les alcools gras, les esters de glycérine d'acide gras et les esters de sorbitane d'acide gras, qui sont chacun éthoxylés et éventuellement propoxylés,
- éventuellement au moins un précurseur de colorant d'oxydation et/ou au moins un colorant direct,
- l'agent présentant un pH compris dans la plage de 7,0 à 8,8, mesuré à 22 °C,
- étant présent sous forme d'hydrogel et
- étant **caractérisé par** une turbidité comprise dans la plage de zéro à 80 NTU, de préférence d'au plus 60 NTU, de manière particulièrement préférée d'au plus 50 NTU, de manière préférée entre toutes d'au plus 40 NTU, à chaque mois mesurée à 22 °C selon DIN EN ISO 7027-C2.

2. Agent selon la revendication 1, **caractérisé en ce qu'**il ne contient pas de tensioactif anionique, ni de tensioactif cationique ni de diéthanolamide d'acides gras.

3. Agent selon la revendication 1 ou 2, **caractérisé en ce que** l'au moins un polyol soluble dans l'eau est choisi parmi au moins un alcanol polyvalent en C₂-C₉ soluble dans l'eau ayant de 2 à 6 groupes hydroxyle et/ou au moins un polyéthylène glycol soluble dans l'eau ayant de 3 à 20 unités d'oxyde d'éthylène ainsi que leurs mélanges, de préférence choisis parmi le 1,2-propylèneglycol, le 1,3-propylèneglycol, le 2-méthyl-1,3-propanediol, la glycérine, le 1,2-butylèneglycol, le 1,3-butylèneglycol, le 1,4-butylèneglycol, le 1,2-pentanediol, le 1,5-pentanediol, le 1,2-hexanediol, le 1,6-hexanediol, le 2-méthyl-2,4-pentanediol, le 1,2,6-hexanetriol, le 1,2-octanediol, le 1,8-octanediol, le dipropylèneglycol, le tripropylèneglycol, la diglycérine, la triglycérine, l'érythritol, le sorbitol, le cis-1,4-diméthylolcyclohexane, le trans-1,4-diméthylolcyclohexane, tout mélange isomère de cis- et de trans-1,4-diméthylolcyclohexane, PEG-3, PEG-4, PEG-6, PEG-7, PEG-8, PEG-9, PEG-10, PEG-12, PEG-14, PEG-16, PEG-18 et PEG-20 ainsi que leurs mélanges.

4. Agent selon l'une des revendications 1 à 3, **caractérisé en ce que** l'au moins un agent alcalinisant est choisi parmi l'ammoniac et au moins une alcanolamine, qui est de préférence choisie parmi le 2-aminoéthane-1-ol (monoéthanolamine), le 3-aminopropane-1-ol, le 4-aminobutane-1-ol, le 5-aminopentane-1-ol, le 1-aminopropane-2-ol (monoisopropanolamine), le 1-amino-butane-2-ol, le 1-aminopentane-2-ol, le 1-aminopentane-3-ol, le 1-aminopentane-4-ol, le 2-amino-2-méthylpropanol, le 2-amino-2-méthylbutanol, le 3-amino-2-méthylpropane-1-ol, le 1-amino-2-méthylpropane-2-ol, le 3-aminopropane-1,2-diol, le 2-amino-2-méthylpropane-1,3-diol, le 2-amino-2-éthyl-1,3-propanediol, la N, N-diméthyl-éthanolamine, la triéthanolamine, la diéthanolamine et la triisopropanolamine, ainsi que des mélanges des substances susmentionnées.

5. Agent selon l'une des revendications 1 à 4, **caractérisé en ce que** l'au moins un solubilisant est choisi parmi les alcanols en C₈-C₂₄ éthoxylés avec de 9 à 100 moles d'oxyde d'éthylène par mole, les produits d'addition d'éthylène glycol d'alkylépoxyde en C₈-C₂₄ qui sont éthoxylés avec de 5 à 20 moles d'oxyde d'éthylène par mole et propoxylés avec 1 mole d'oxyde de propylène par mole, les mono-, di- ou triesters de glycérine d'acides carboxyliques en C₁₂-C₃₀ linéaires saturés et insaturés éthoxylés avec de 9 à 100 moles d'oxyde d'éthylène par mole, qui peuvent être hydroxylés, en particulier ceux d'acide myristique, d'acide palmitique, d'acide stéarique, d'acide oléique ou de mélanges de ces acides gras, les acides carboxyliques en C₈-C₂₄ éthoxylés avec de 9 à 100 moles d'oxyde d'éthylène par mole, les monoesters de sorbitane d'acides carboxyliques en C₁₂-C₃₀ linéaires saturés et insaturés éthoxylés avec de 9 à 100 moles d'oxyde d'éthylène par mole, qui peuvent être hydroxylés, en particulier ceux d'acide myristique, d'acide palmitique, d'acide stéarique, d'acide oléique ou de mélanges de ces acides gras, ainsi que des mélanges des substances susmentionnées.

6. Agent selon l'une des revendications 1 à 5, **caractérisé en ce que** l'au moins un solubilisant est choisi parmi au moins un composé de nom INCI PPG-1-PEG-9 Lauryl Glycol Ether.

7. Agent prêt à l'utilisation pour blanchir et/ou colorer les fibres kératiniques, consistant en un mélange de
25 à 75 % en poids, de préférence de 40 à 60 % en poids, de manière particulièrement préférée de 50 % en poids d'un agent selon l'une des revendications 1 à 6 et
75 à 25 % en poids, de préférence de 60 à 40 % en poids, de manière particulièrement préférée de 50 % en poids d'une préparation d'agent oxydant, contenant
- 75 à 95% en poids, par rapport au poids de la préparation d'agent oxydant, d'eau ou d'un mélange d'eau et d'au moins un polyol soluble dans l'eau, de préférence choisi parmi au moins un alcanol polyvalent en C₂ à C₉ soluble dans l'eau ayant de 2 à 6 groupes hydroxyle et/ou au moins un polyéthylène glycol soluble dans l'eau ayant de 3 à 20 unités d'oxyde d'éthylène ainsi que leurs mélanges, de manière particulièrement préférée choisis parmi le propylèneglycol, le 1,3-propylèneglycol, le 2-méthyl-1,3-propanediol, la glycérine, le 1,2-butylèneglycol, le 1,3-butylèneglycol, le 1,4-butylèneglycol, le 1,2-pentanediol, le 1,5-pentanediol, le 1,2-hexanediol, le 1,6-hexanediol, le 2-méthyl-2,4-pentanediol, le 1,2,6-hexanetriol, le 1,2-octanediol, le 1,8-octanediol, le dipropylèneglycol, le tripropylèneglycol, la diglycérine, la triglycérine, l'érythritol, le sorbitol, le cis-1,4-diméthylolcyclohexane, le trans-1,4-diméthylolcyclohexane, tout mélange isomérique de cis- et de trans-1,4-diméthylcyclohexane, PEG-3, PEG-4, PEG-6, PEG-7, PEG-8, PEG-9, PEG-10, PEG-12, PEG-14, PEG-16, PEG-18 et PEG-20 ainsi que leurs mélanges, mais aussi
- au moins un polymère formant un hydrogel choisi dans le groupe comprenant les polysaccharides, les polymères et les copolymères d'acide acrylique, les polymères et les copolymères d'acide méthacrylique et les polymères et les copolymères d'acide 2-méthyl-2[(1-oxo-2-propényl)amino]-1-propane-sulfonique, ainsi que leurs mélanges, mais aussi
- de 1,0 à 23,0 % en poids de peroxyde d'hydrogène, par rapport au poids de la préparation d'agent oxydant,
- la préparation d'agent oxydant présentant un pH, à chaque fois mesuré à 22 °C, situé dans la plage de 2,0 à 6,5, de préférence dans la plage de 3,0 à 4,5.

8. Agent prêt à l'utilisation selon la revendication 7, **caractérisé par** un pH compris dans la plage de 6,0 à 7,3, de préférence de 6,2 à 6,9, à chaque fois mesuré à 22 °C.

9. Kit de coloration et/ou d'éclaircissement des fibres kératiniques, comprenant un agent selon l'une quelconque des revendications 1 à 6 et une préparation d'agent oxydant, contenant
- de 75 à 93 % en poids, par rapport au poids de la préparation d'agent oxydant, d'eau ou d'un mélange d'eau et d'au moins un polyol soluble dans l'eau, de préférence choisi parmi au moins un alcanol polyvalent en C₂ à C₉ soluble dans l'eau ayant de 2 à 6 groupes hydroxyle et/ou au moins un polyéthylène glycol soluble dans l'eau ayant de 3 à 20 unités d'oxyde d'éthylène ainsi que leurs mélanges, de manière particulièrement préférée choisis parmi le 1,2-propylèneglycol, le 1,3-propylèneglycol, le 2-méthyl-1,3-propanediol, la glycérine, le 1,2-butylèneglycol, le 1,3-butylèneglycol, le 1,4-butylèneglycol, le 1,2-pentanediol, le 1,5-pentanediol, le 1,2-hexanediol, le 1,6-hexanediol, le 2-méthyl-2,4-pentanediol, le 1,2,6-hexanetriol, le 1,2-octanediol, le 1,8-octanediol, le dipropylèneglycol, le tripropylèneglycol, la diglycérine, la triglycérine, l'érythritol, le sorbitol, le cis-1,4-diméthylolcyclohexane, le trans-1,4-diméthylolcyclohexane, tout mélange isomérique de cis- et de trans-1,4-diméthylcyclohexane, PEG-3, PEG-4, PEG-6, PEG-7, PEG-8, PEG-9, PEG-10, PEG-12, PEG-14, PEG-16, PEG-18 et PEG-20 ainsi que leurs mélanges, mais aussi
- au moins un polymère formant un hydrogel choisi dans le groupe comprenant les polysaccharides, les polymères et les copolymères d'acide acrylique, les polymères et les copolymères d'acide méthacrylique et les polymères et les copolymères d'acide 2-méthyl-2[(1-oxo-2-propényl)amino]-1-propane-sulfonique, ainsi que leurs mélanges, mais aussi
- de 1,0 à 23 % en poids de peroxyde d'hydrogène, par rapport au poids de la préparation d'agent oxydant,
- la préparation d'agent oxydant présentant un pH, à chaque fois mesuré à 22 °C, situé dans la plage de 2,0 à 6,5, de préférence dans la plage de 3,0 à 4,5.

10. Procédé de coloration et/ou d'éclaircissement des fibres kératiniques, **caractérisé en ce que**
- éventuellement, un agent de prétraitement M1 est appliqué sur les fibres, puis
- un agent prêt à l'utilisation M2 selon la revendication 7 ou 8 est produit et appliqué sur les fibres,
- cet agent M2 est rincé de la fibre au bout d'une durée de 1 à 60 minutes
- et ensuite, éventuellement, un agent de post-traitement M3 est appliqué sur la fibre et rincé à nouveau après un temps de pose de 0,5 à 30 minutes.
